# EUROPEAN PATENT APPLICATION

(11) **EP 3 831 419 A1**
(43) Date of publication of application: **09.06.2021**
(21) Application number: 19843124.9
(22) Date of filing: 31.07.2019
(51) Int. Cl.: A61L 27/38, A61L 27/36, A61L 27/56, A61L 27/52

(54) **METHOD FOR MANUFACTURING MULTILAYERED CELL SHEET, AND MULTILAYERED CELL SHEET MANUFACTURED USING SAME**

(30) Priority: 31.07.2018 KR 20180089461
(71) Applicant: Rokit Healthcare Inc., Geumcheon-gu Seoul 08512 (KR)
(72) Inventor: YOU, Seok Hwan, Seoul 06943 (KR); KIM, Jae Yun, Bucheon-si Gyeonggi-do 14548 (KR)
(74) Representative: Maiwald Patent- und Rechtsanwaltsgesellschaft mbH
(86) International application number: PCT/KR2019/009544
(87) International publication number: WO 2020/027575

(57) **Abstract**

The present specification relates to a method for manufacturing a multilayered cell sheet, and a multilayered cell sheet manufactured using same, the method comprising the steps of: (a) forming a first cell layer on a first substrate, which has a melting point or is changed from being hydrophobic to being hydrophilic at any one temperature from 0°C to 30°C; (b) forming a second cell layer on a second substrate to be degraded by an enzyme; (c) making the first cell layer and the second cell layer come in contact with each other; (d) selectively removing the first substrate by providing a temperature lower than or equal to the melting point of the first substrate or a temperature at which the first substrate is changed to being hydrophilic; and (e) selectively removing the second substrate by making the second substrate come in contact with a solution containing the enzyme.

## Description

### [Technical Field]

The present specification claims priority to and the benefit of Korean Patent Application No. 10-2018-0089461 filed in the Korean Intellectual Property Office on July 31, 2018, the entire contents of which are incorporated herein by reference.

The present invention relates to the fields of tissue engineering and regenerative medicine, and specifically to a method for manufacturing a multilayered cell sheet and a multilayered cell sheet manufactured using the same.

### [Background Art]

Tissue engineering treatment in the regenerative medicine field is evolving toward culturing cells on a biodegradable polymer support to reconstruct a tissue, and then transplanting the tissue into a damaged site to induce a normal function. However, when the biodegradable polymer support is transplanted into the body, there is a problem in that an inflammatory reaction occurs due to the production of an acidic material (Ronneberger B et al., J Biomed Mater Res, 30 (1) (1996), 31-40).

As another approach, a method of mixing cells with a biodegradable polymer solution and injecting the mixed solution into a damaged site has been proposed, but the method has a problem in that the cell regeneration efficiency at a transplanted site greatly deteriorates due to the damage of an extracellular matrix (ECM) (Canavan H et al. J Biomed Mater Res A. 2005 Oct 1;75(1):1-13).

In order to solve the above problems, a cell sheet has been developed as a means for transplanting cells without a biodegradable polymer (Yang J et al. Biomaterials. 2005 Nov;26(33):6415-22). However, even though a cell sheet is manufactured, a process of stacking the cell sheet in multiple layers is required for clinical application, but currently known methods have a problem in that the methods are not economically feasible due to a complicated process and a long manufacturing time.

### [Disclosure]

### [Technical Problem]

The present invention has been made in an effort to provide a method for manufacturing a cell sheet having a multilayered structure by a simple process, and a multilayered cell sheet manufactured using the same.

### [Technical Solution]

An exemplary embodiment of the present invention provides a method for manufacturing a multilayered cell sheet, the method comprising the steps of: (a) forming a first cell layer on a first substrate, which has a melting point or is changed from being hydrophobic to being hydrophilic at any one temperature from 0°C to 30°C; (b) forming a second cell layer on a second substrate to be degraded by an enzyme; (c) making the first cell layer and the second cell layer come in contact with each other; (d) selectively removing the first substrate by providing a temperature lower than or equal to the melting point of the first substrate or a temperature at which the first substrate is changed to being hydrophilic; and (e) selectively removing the second substrate by making the second substrate come in contact with a solution containing the enzyme.

Another exemplary embodiment of the present invention provides a multilayered cell sheet manufactured using the manufacturing method.

### [Advantageous Effects]

The multilayered cell sheet according to the present invention has an advantage in that a multilayered cell sheet can be manufactured by a simple and economical method.

### [Description of Drawings]

FIG. 1 illustrates a method for manufacturing a multilayered cell sheet according to an exemplary embodiment of the present invention.
FIG. 2 illustrates an enlarged image of a Pluronic hydrogel sheet according to the Example.
FIG. 3 illustrates an enlarged image of the cell sheet on a surface from which the Pluronic hydrogel sheet in the Example has been removed.
FIG. 4 illustrates a result of confirming whether cells on the cell sheet on a surface from which the Pluronic hydrogel sheet in the Example has been removed survive through staining.
FIG. 5 illustrates an enlarged image of the cell sheet on a surface from which an alginate hydrogel sheet in the Example has been removed.
FIG. 6 illustrates a result of confirming whether cells on the cell sheet on a surface from which the alginate hydrogel sheet in the Example has been removed survive through staining.
FIG. 7 illustrates a photograph of a cell sheet from which both the Pluronic hydrogel sheet and the alginate hydrogel sheet according to the Example have been removed.

### [Modes of the Invention]

When one member is disposed "on" another member in the present specification, this includes not only a case where the one member is brought into contact with another member, but also a case where still another member is present between the two members.

When one part "includes" one constituent element in the present specification, unless otherwise specifically described, this does not mean that another constituent element is excluded, but means that another constituent element may be further included.

In the present specification, the "additional first substrate" may include all the content on a first substrate, and may also be the same as the first substrate.

In the present specification, the "additional second substrate" may include all the content on a second substrate, and may also be the same as the second substrate.

In the present specification, the "additional first cell layer" may include all the content on a first cell layer, and may also be the same as the first cell layer.

In the present specification, the "additional second cell layer" may include all the content on a second cell layer, and may also be the same as the second cell layer.

Hereinafter, the present invention will be described in detail.

An exemplary embodiment of the present invention provides a method for manufacturing a multilayered cell sheet, the method comprising the steps of:
(a) forming a first cell layer on a first substrate, which has a melting point or is changed from being hydrophobic to being hydrophilic at any one temperature from 0°C to 30°C;
(b) forming a second cell layer on a second substrate to be degraded by an enzyme;
(c) making the first cell layer and the second cell layer come in contact with each other;
(d) selectively removing the first substrate by providing a temperature lower than or equal to the melting point of the first substrate or a temperature at which the first substrate is changed to being hydrophilic; and
(e) selectively removing the second substrate by making the second substrate come in contact with a solution containing the enzyme.

The method for manufacturing a multilayered cell sheet according to the present invention has an advantage in that a cell sheet in which multilayered cell layers are stacked can be manufactured by a simple method. The method for manufacturing a multilayered cell sheet may stack a cell layer using: a first substrate, which has a melting point or is changed from being hydrophobic to being hydrophilic at any one temperature from 0°C to 30°C; and a second substrate to be degraded by an enzyme, and selectively remove any one substrate. An additional cell layer may be stacked on a cell layer to be exposed by the selectively removed substrate using the first substrate and the second substrate. In addition, since the stacked cell layer is provided on the substrate which has not been removed, the cell layer may be easily transferred, and after the transfer, the remaining substrate may be selectively removed to transplant the multilayered cell layer.

According to an exemplary embodiment of the present invention, the steps of: (d) and (e) are sequentially performed, and it is possible to further include a step of (d') selectively removing an additional first substrate by making the exposed first layer and an additional first cell layer provided on the additional first substrate come in contact with each other, and then providing a temperature lower than or equal to the melting point of the additional first substrate or a temperature at which the first additional substrate is changed to being hydrophilic between the steps of: (d) and (e). Specifically, as a stacked structure body of the "first substrate/first cell layer/second cell layer/second substrate" formed by the step of (c), a stacked structure body of an "additional first cell layer/first cell layer/second cell layer/second substrate" may be formed by the steps of: (d) and (d').

Furthermore, according to an exemplary embodiment of the present invention, the step of (d') may be repeatedly performed two or more times to form a plurality of additional first cell layers on the first cell layer. Specifically, the step of (d') may be repeatedly performed multiple times, thereby allowing a stacked structure body of "additional first cell layer/.../ additional first cell layer/first cell layer/second cell layer/second substrate" to be formed. The number of additional first cell layers may be appropriately adjusted according to the use and purpose of the multilayered cell sheet.

According to an exemplary embodiment of the present invention, the steps of: (e) and (d) are sequentially performed, and it is possible to further include a step of (e') selectively removing an additional second substrate by making the exposed second cell layer and an additional second cell layer provided on the additional second substrate come in contact with each other, and then making the additional second substrate come in contact with a solution containing the enzyme between the steps of: (e) and (d). Specifically, as a stacked structure body of the "first substrate/first cell layer/second cell layer/second substrate" formed by the step of (c), a stacked structure body of a "first substrate/first cell layer/second cell layer/additional second cell layer" may be formed by the steps of: (e) and (d').

Further, according to an exemplary embodiment of the present invention, the step of (e') may be repeatedly performed two or more times to form a plurality of additional second cell layers on the second cell layer. Specifically, the step of (e') may be repeatedly performed multiple times, thereby a stacked structure body of "first substrate/first cell layer/second cell layer/additional second cell layer/.../additional second cell layer" to be formed. The number of additional second cell layers may be appropriately adjusted according to the use and purpose of the multilayered cell sheet.

According to an exemplary embodiment of the present invention, the first substrate may have a solid phase or hydrophobicity in a temperature atmosphere more than 30°C. Specifically, the first substrate may be converted into a liquid phase or hydrophilic at a temperature of 30°C or less, and may be selectively removed from the first cell layer using the conversion.

According to an exemplary embodiment of the present invention, the steps of: (a) and (c) may be performed in an atmosphere of 35°C to 40°C, specifically in an atmosphere of 36°C to 38°C, and more specifically in an atmosphere of 36°C to 37°C. Since the first substrate has a melting point or is changed from being hydrophobic to being hydrophilic at any one temperature from 0°C to 30°C, this is for facilitating the formation of a cell layer by allowing the first substrate to maintain a solid phase form or hydrophobicity and allowing cells to become active, in an atmosphere in the temperature range (that is, 35°C to 40°C). Likewise, since the additional first substrate in the step of (d') has the same properties as the first substrate, the process of forming the additional first cell layer and stacking the additional first cell layer may be performed in an atmosphere of 35°C to 40°C. Furthermore, the step of (b) may also be performed in an atmosphere of 35°C to 40°C, specifically in an atmosphere of 36°C to 38°C, and more specifically in an atmosphere of 36°C to 37°C in order to facilitate the formation of the cell layer.

According to an exemplary embodiment of the present invention, the step of (c) may be making the first cell layer and the second cell layer come in contact with each other by pressurizing the layers. In this case, the pressurization condition may be appropriately adjusted such that the cell layers to be in contact with each other are brought into close contact to enable the exchange of the material without being destroyed. Likewise, the additional first cell layer and the additional second cell layer in the steps of: (d') and (e') may be pressurized and stacked on the cell layer.

According to an exemplary embodiment of the present invention, the first substrate, the second substrate, the additional first substrate, and the additional second substrate may each be a hydrogel substrate containing hydrogel as a main component.

The "hydrogel" is a material that may contain a large amount of water, and may refer to a material or form capable of easily delivering and moving materials required for cell survival such as oxygen, water, water-soluble nutrients, polypeptides such as enzymes and cytokines, and waste products, and the like. The hydrogel may be hydrogel particles formed by solidifying an aqueous solution containing colloidal particles. The hydrogel may be particles including hydrogels obtained by chemically cross-linking, for example, a water-soluble, hydrophilic or water-absorbing synthetic polymer such as poly(acrylamide), poly(acrylic acid), poly(hydroxyethyl methacrylate), poly(vinyl alcohol), poly(lactic acid), and poly(glycolic acid); and a polysaccharide, a protein, and a nucleic acid, and the like. Examples of the polysaccharide include glycosaminoglycans such as hyaluronic acid and chondroitin sulfate, starch, glycogen, agar, pectin, fibrin and the like, but are not limited thereto. Further, examples of the protein include collagen and its hydrolysate gelatin, a proteoglycan, fibronectin, vitronectin, laminin, entactin, tenascin, thrombospondin, a von Willebrand factor, osteopontin, fibrinogen, and the like, but are not limited thereto.

According to an exemplary embodiment of the present invention, the first substrate may contain at least one of a polyphosphazene-based hydrogel, a Pluronic-based hydrogel, and poly(N-isopropylacrylamide).

Specifically, the first substrate and the additional first substrate may each be a polyphosphazene-based substrate, a Pluronic-based substrate, or a poly(N-isopropylacrylamide) substrate. In addition, the additional first substrate may be the same as the content of the first substrate.

According to an exemplary embodiment of the present invention, the polyphosphazene-based hydrogel contains a temperature-sensitive polyphosphazene-based compound, so that the melting point thereof may be adjusted from about 4°C to about 10°C. For example, a temperature-sensitive and cross-linkable phosphazene-based hydrogel disclosed in Korean Patent Application Laid-Open No. 10-2017-0061530, a phosphazene-based polymer having an ionic group whose degradation rate can be adjusted, which is disclosed in Korean Patent Application Laid-Open No. 10-2014-0016521, and a biodegradable temperature-sensitive polyphosphazene-based hydrogel disclosed in Korean Patent Application Laid-Open No. 10-2007-0076386 may be included in the polyphosphazene-based hydrogel of the present invention.

According to an exemplary embodiment of the present invention, the melting point temperature of the Pluronic-based hydrogel may be adjusted from about 0°C to 30°C using a Pluronic polymer. The Pluronic polymer can be used as long as it is a polymer having a polyethylene oxide (PEO)-polypropylene oxide (PPO)-polyethylene oxide (PEO) structure (PEO-PPO-PEO). For example, F38, F68, F77, F98, F108, and F127 derivatives starting with F, and the like, L31, L42, L43, L44, L62, L72, and L101 derivatives starting with L, and the like, and P75, P103, and P104 derivatives starting with P, and the like (all are trade names) may be included. More specifically, among the Pluronic polymers, F68 having a molecular weight of about 8,700 daltons and F127 having a molecular weight of about 12,600 daltons approved by the US Food and Drug Administration (FDA) may be used.

However, the first substrate is not limited to the polyphosphazene-based hydrogel and the Pluronic-based hydrogel, and any hydrogel may be applied as the first substrate and/or the additional first substrate as long as it is a hydrogel having a melting point at a temperature of 30°C or less.

In addition, according to an exemplary embodiment of the present invention, the first substrate may be a poly(N-isopropylacrylamide) substrate. Furthermore, the first substrate and/or the additional first substrate may be surface-treated with poly(N-isopropylacrylamide). Specifically, the surface of the first substrate and/or the additional first substrate may be kraft-bonded with poly(N-isopropylacrylamide) or the first substrate and/or the additional first substrate may be surface-coated with poly(N-isopropylacrylamide). Since the poly(N-isopropylacrylamide) is converted from being hydrophobic to being hydrophilic in an atmosphere of 30°C or less, specifically in an atmosphere of about 20°C to about 30°C, the first substrate may be easily and selectively removed using a physiological saline solution at about 20°C to about 30°C when the first substrate is a poly(N-isopropylacrylamide) substrate or a substrate surface-treated with the poly(N-isopropylacrylamide). Further, through the poly(N-isopropylacrylamide), a cell layer may be better formed on the first substrate and/or the additional first substrate, and when the first substrate and/or the additional first substrate are/is removed, the poly(N-isopropylacrylamide) may also be easily separated from the cell layer. However, the present invention is not limited thereto, and any material having a property of being changed from being hydrophobic to being hydrophilic at a temperature of 30°C or less can be applied in the same manner as the poly (N-isopropylacrylamide).

According to an exemplary embodiment of the present invention, the step of (d) may make the first substrate come in contact with a solution having a temperature lower than or equal to the melting point of the first substrate or a temperature at which the first substrate is changed to being hydrophilic, or allow the ambient temperature to be lowered to a temperature lower than or equal to the melting point of the first substrate or a temperature at which the first substrate is changed to being hydrophilic. Specifically, the process of selectively removing the first substrate in the step of (d) may selectively remove the first substrate by immersing the stacked structure body of "first substrate/first cell layer/second cell layer/second substrate" formed by the step of (c) into a solution having a temperature lower than or equal to the melting point of the first substrate or a temperature at which the first substrate is changed to being hydrophilic. In addition, the process of selectively removing the first substrate in the step of (d) may selectively remove the first substrate by lowering the ambient temperature (atmospheric temperature) of the stacked structure body of "first substrate/first cell layer/second cell layer/second substrate" formed by the step of (c) to a temperature lower than or equal to the melting point of the first substrate or a temperature at which the first substrate is changed to being hydrophilic. In this case, the solution having a temperature lower than or equal to the melting point of the first substrate may be a physiological saline solution having a temperature of about 4°C to about 30°C. Furthermore, the ambient temperature (atmospheric temperature) may be about 4°C to about 30°C. Likewise, the process of selectively removing the additional first substrate in the step of (d') may selectively remove the additional first substrate by immersing the stacked structure body into a physiological saline solution having a temperature of about 4°C to about 30°C, or lowering the ambient temperature (atmospheric temperature) to a temperature lower than or equal to the melting point of the first substrate or a temperature at which the first substrate is changed to being hydrophilic. When the cell layer of the stacked structure is likely to be damaged in a solution having a temperature lower than or equal to the melting point of the first substrate or the additional first substrate, or at an ambient temperature lower than 20°C, the first substrate may be selectively removed at 20°C to 30°C using a poly(N-isopropylacrylamide) substrate or a substrate surface-treated with poly(N-isopropylacrylamide).

According to an exemplary embodiment of the present invention, the second substrate may be a hydrogel substrate containing an enzyme-susceptible peptide. For example, the second substrate may be a hydrogel containing an enzyme-susceptible peptide and a poly(ethylene glycol) (PEG) gel. Further, the second substrate may be a hydrogel substrate to which an amino acid sequence capable of being degraded by matrix metalloproteinases (MMPs), elastase and/or plasmin and/is attached. Specifically, the second substrate may be a PEG-succinimidyl propionate hydrogel substrate to which an amino acid sequence is attached, for example, a PEG-succinimidyl propionate hydrogel substrate to which a PEG-amine functionalized with a synthetic tetrapeptide Ala-Pro-Gly-Leu (4armPEG10k-LGPA) is attached. Alternatively, the second substrate may be a PEG-hydrogel substrate to which an amine reactive PEG-monoacrylate and a collagenase sensitive peptide)(Gly-Gly-Leu'Gly-Pro-Ala-Gly-Gly-Lys), or an integrin-binding domain peptide (Tyr-Ile-Shy-Ser-Arg) is attached.

According to an exemplary embodiment of the present invention, the second substrate may be a hydrogel substrate containing carboxymethyl cellulose (CMC) or alginate (Al).

Specifically, according to an exemplary embodiment of the present invention, the carboxymethyl cellulose or alginate may be conjugated with tyramine. That is, the second substrate and/or the additional second substrate may be a hydrogel substrate containing carboxymethyl cellulose conjugated with tyramine (CMC-ty). Furthermore, the second substrate and/or the additional second substrate may be a hydrogel substrate containing alginate conjugated with tyramine (Al-ty).

The second substrate and/or the additional second substrate may be a hydrogel substrate containing at least 0.5% CMC-ty or Al-ty. Specifically, the second substrate and/or the additional second substrate may be a hydrogel substrate containing 0.5% to 4% CMC-ty or Al-ty. The % may be wt% or vol%.

According to an exemplary embodiment of the present invention, the enzyme may be a carboxymethyl cellulose-degrading enzyme or an alginate-degrading enzyme. Specifically, when the second substrate and/or the additional second substrate are/is a carboxymethyl cellulose substrate, the enzyme may be a carboxymethyl cellulose-degrading enzyme. Further, when the second substrate and/or the additional second substrate are/is an alginate substrate, the enzyme may be an alginate-degrading enzyme.

The enzyme may be removed by selectively degrading the second substrate and/or the additional second substrate in the steps of: (e) and/or (e'). In addition, the enzyme may not degrade the remaining components (for example, the first substrate, the first cell layer, the second cell layer, and the like) except for the second substrate and the additional second substrate. Specifically, the enzyme may not degrade the cells and extracellular matrix of the first cell layer and the second cell layer.

According to an exemplary embodiment of the present invention, the first substrate and the second substrate may have a predetermined pattern suitable for implementing the characteristics of the first cell layer and the second cell layer, respectively. Specifically, the pattern of a required substrate may vary according to the types of cells constituting each of the cell layers, and the culture and characteristics of cells may be well implemented on a substrate provided with a pattern suitable for the cells. For example, when a cell layer is formed using muscle cells, the muscle cell layer may be formed faster on a substrate having a pattern similar to that of a muscle tissue, and the characteristics of muscle cells may be more easily expressed.

Therefore, the first substrate and the second substrate may include a predetermined pattern such that the characteristics of a cell layer to be cultured are better implemented. When the first substrate and the second substrate have a predetermined pattern, the first substrate and the second substrate may include an uneven pattern on one surface thereof, or may have various thicknesses according to the predetermined pattern over the entire substrate. As a method for patterning the first substrate and/or the second substrate, a method known in the art may be used. For example, patterning may be performed using soft lithography, self-assembly, vapor deposition, photolithography, and the like.

According to an exemplary embodiment of the present invention, the first substrate and the second substrate may have a predetermined strength suitable for implementing the characteristics of the first cell layer and the second cell layer, respectively. Specifically, the strength (modulus) of a required substrate may vary according to the types of cells constituting each of the cell layers, and the culture and characteristics of cells may be well implemented on a substrate having a strength suitable for the cells.

According to an exemplary embodiment of the present invention, the first cell layer and the second cell layer may be formed by being cultured on the first substrate and the second substrate, respectively. Likewise, the additional first cell layer may be formed by being cultured on the additional first substrate, and the additional second cell layer may be cultured by being cultured on the additional substrate.

According to an exemplary embodiment of the present invention, the first cell layer and the second cell layer may each include cells selected from the group consisting of mesenchymal stem cells (MSCs), myocyte precursor cells, myocytes, fibroblasts, chondrocytes, endothelial cells, epithelial cells, embryonic stem cells (ESCs), hematopoietic stem cells, anchorage-dependent cell precursors, induced pluripotent stem cells (iPSCs), and cardiomyocytes. Specifically, the first cell layer, the second cell layer, the additional first cell layer, and the additional second cell layer may be formed by culturing at least one of the above cells. Furthermore, the first cell layer and the second cell layer may be those in which the same cells are cultured, or those in which cells different from each other are cultured.

Another exemplary embodiment of the present invention provides a multilayered cell sheet manufactured using the manufacturing method. The multilayered cell sheet may implement a three-dimensional structure similar to a site to be transplanted. Specifically, since the multilayered cell sheet may be manufactured by stacking a cell layer formed using a substrate having a predetermined pattern, the multilayered cell sheet may have a three-dimensional structure having a form similar to that of a target tissue. Through this, the multilayered cell sheet has a high cell survival rate after transplantation, and further has an advantage of being effective for tissue regeneration.

Further, since the multilayered cell sheet may implement a structure and characteristics similar to those of a human tissue, the multilayered cell sheet may be used for drug testing. Specifically, the multilayered cell sheet may be used in order to examine a tissue response to a new drug.

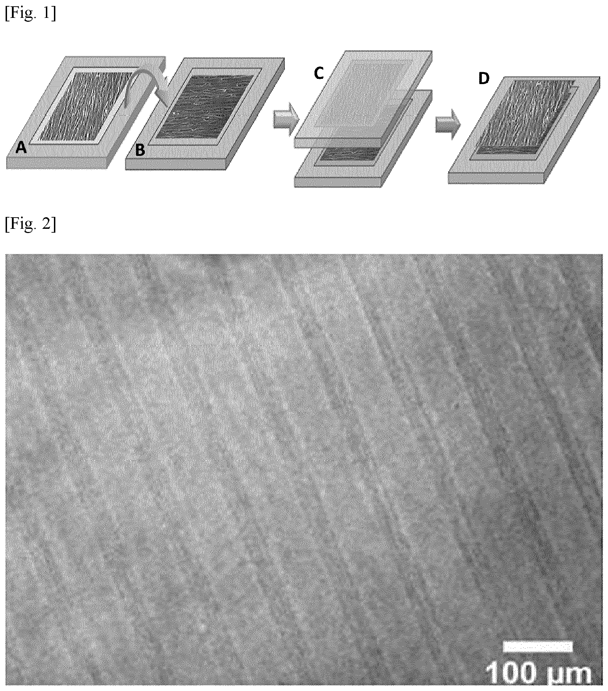

FIG. 1 illustrates a method for manufacturing a multilayered cell sheet according to an exemplary embodiment of the present invention. Specifically, FIG. 1 illustrates that after each cell layer of the first substrate (A) on which the first cell layer has been formed and the second substrate (B) on which the second cell layer has been formed is made to come in contact with each other (C), a cell sheet provided with a two-layered cell layer is manufactured by lowering the temperature to remove the first substrate. As a subsequent step in FIG. 1, additional cell layers may be further stacked, or a cell sheet including a two-layered cell layer may also be obtained by removing the second substrate. However, the present invention is not limited to FIG. 1, and may further include additional configurations and/or steps.

Hereinafter, the present specification will be described in detail with reference to Examples for specifically describing the present specification. However, the Examples according to the present specification may be modified into various forms, and it is not to be interpreted that the scope of the present specification is limited to the Examples described below in detail. The Examples of the present specification are provided to describe the present specification more completely to a person with ordinary skill in the art.

### [Examples]

### Manufacture of Pluronic hydrogel sheet (first substrate)

A gelatin mold was prepared to manufacture a hydrogel sheet, and a micropattern was formed on the bottom of the mold. An aqueous solution of about 10 wt% Pluronic F-127 (Sigma) was put into the gelatin mold in an atmosphere of about 25°C. And, a nitrocellulose film was used as a cover to flatten the upper surface of the manufactured hydrogel sheet, and a plastic mesh and a glass plate for fixing the nitrocellulose film were stacked. And then, only the aqueous solution of Pluronic F-127 (Sigma) in the mold was cured and subjected to hydrogelation by increasing the temperature to about 37°C. Thereafter, a Pluronic hydrogel sheet was manufactured by removing the gelatin mold by a method of dissolving the gelatin mold using a washing buffer (Saline, PBS, ddH ₂O) at about 37°C.

FIG. 2 illustrates an enlarged image of a Pluronic hydrogel sheet according to the Example. Since the Pluronic hydrogel sheet is transparent, the formed pattern can be confirmed through a bright field image using an electron microscope as illustrated in FIG. 2. As confirmed in FIG. 2, it can be confirmed that the manufactured Pluronic hydrogel sheet has a stripe pattern formed at certain intervals.

### Manufacture of alginate hydrogel sheet (second substrate)

A gelatin mold was prepared to manufacture a hydrogel sheet, and a micropattern was formed on the bottom of the mold . In an atmosphere of about 25°C, an alginate solution containing alginate at a content of about 2 wt% in a MES buffer having a pH of 6 to 7 was put into the gelatin mold. And, a nitrocellulose film was used as a cover to flatten the upper surface of the manufactured hydrogel sheet, and a plastic mesh and a glass plate for fixing the nitrocellulose film were stacked. And then, the alginate solution in the mold was subjected to hydrogelation by adding an alginate cross-linking aqueous solution dropwise thereto. Thereafter, an alginate hydrogel sheet was manufactured by removing the gelatin mold by a method of dissolving the gelatin mold using a washing buffer (Saline, PBS, ddH ₂O) at about 37°C.

### Manufacture of multilayered cell sheet

In an atmosphere of about 37°C, a cell layer was formed by culturing C2C12 skeletal muscle cells on the surface of the manufactured Pluronic hydrogel sheet on which a pattern was formed, and a cell layer was formed by culturing C2C12 skeletal muscle cells on a surface of the alginate hydrogel sheet. And then, after the cell layers were made to come in contact with each other, the Pluronic hydrogel sheet was dissolved and removed by lowering the temperature to about 10°C, thereby manufacturing a 2-stack cell sheet. And then, the temperature was again increased to about 37°C to activate the cell layer.

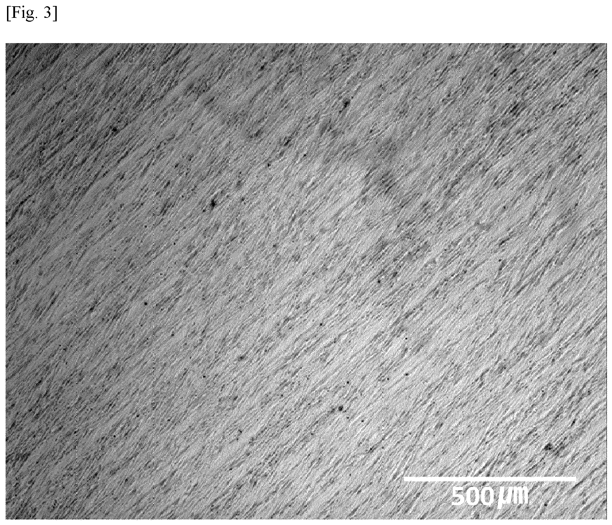

FIG. 3 illustrates an enlarged image of the cell sheet on a surface from which the Pluronic hydrogel sheet in the Example has been removed. Specifically, FIG. 3 is a captured bright field image of the surface from which the Pluronic hydrogel sheet has been removed using an electron microscope.

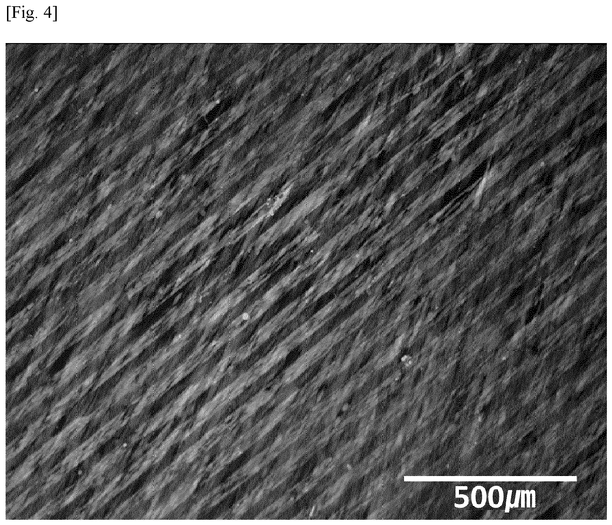

FIG. 4 illustrates a result of confirming whether cells on the cell sheet on a surface from which the Pluronic hydrogel sheet in the Example has been removed survive through staining. Specifically, the staining in FIG. 4 uses a Live/Dead mammalian cell kit from Thermo Fisher, and live cells are stained with calcein AM and stained green, and dead cells are stained with ethidium homodimer-1 and shown to be stained red. As can be confirmed in FIG. 4, it can be confirmed that 99% or more of the cells are alive even though the Pluronic hydrogel sheet has been removed.

Furthermore, the alginate hydrogel sheet was removed using a solution containing an alginate-degrading enzyme.

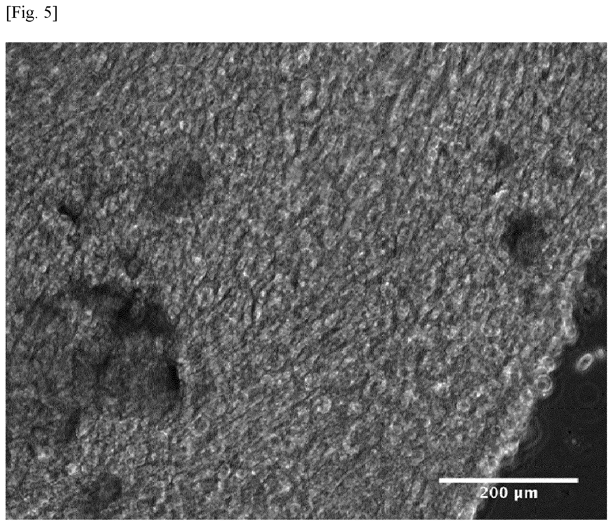

FIG. 5 illustrates an enlarged image of the cell sheet on a surface from which an alginate hydrogel sheet in the Example has been removed. Specifically, FIG. 5 is a captured bright field image of the surface from which the alginate hydrogel sheet has been removed using an electron microscope. In FIG. 5, it is confirmed that a partially recessed region is a partially contracted region of the cell sheet while the alginate hydrogel sheet is separated from the cell sheet.

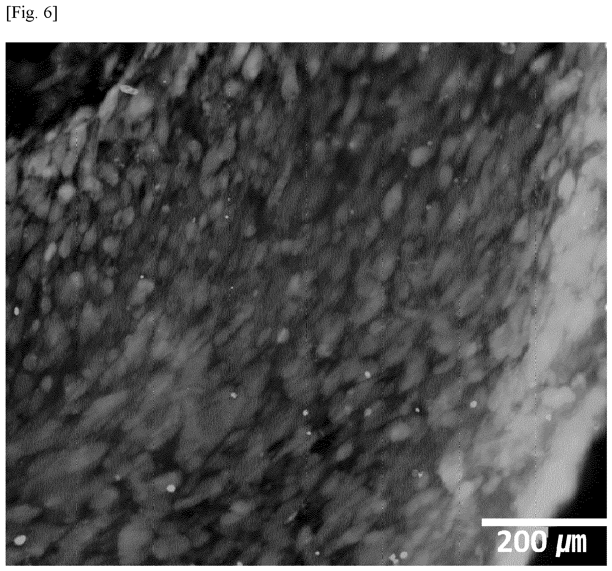

FIG. 6 illustrates a result of confirming whether cells on the cell sheet on a surface from which the alginate hydrogel sheet in the Example has been removed survive through staining. Specifically, the staining in FIG. 6 uses a Live/Dead mammalian cell kit from Thermo Fisher, and live cells are stained with calcein AM and stained green, and dead cells are stained with ethidium homodimer-1 and shown to be stained red. As can be confirmed in FIG. 6, it can be confirmed that 99% or more of the cells are alive even though the alginate hydrogel sheet has been removed.

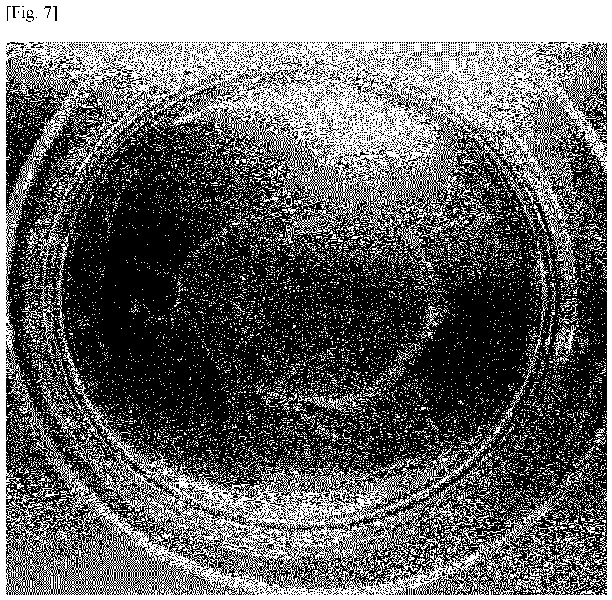

FIG. 7 illustrates a photograph of a cell sheet from which both the Pluronic hydrogel sheet and the alginate hydrogel sheet according to the Example have been removed. As can be confirmed in FIGS. 4, 6, and 7, it can be seen that the cell sheet produced as in the Example can be manufactured while showing high viability in its intact form.

## Claims

1. A method for manufacturing a multilayered cell sheet, the method comprising the steps of: (a) forming a first cell layer on a first substrate, which has a melting point or is changed from being hydrophobic to being hydrophilic at any one temperature from 0°C to 30°C; (b) forming a second cell layer on a second substrate to be degraded by an enzyme; (c) making the first cell layer and the second cell layer come in contact with each other; (d) selectively removing the first substrate by providing a temperature lower than or equal to the melting point of the first substrate or a temperature at which the first substrate is changed to being hydrophilic; and (e) selectively removing the second substrate by making the second substrate come in contact with a solution containing the enzyme.

2. The method of claim 1, wherein the steps of: (d) and (e) are sequentially performed, and further comprising a step of (d') selectively removing an additional first substrate by making the exposed first layer and an additional first cell layer provided on the additional first substrate come in contact with each other, and then providing a temperature lower than or equal to the melting point of the additional first substrate or a temperature at which the additional first substrate is changed to being hydrophilic between the steps of: (d) and (e).

3. The method of claim 2, wherein the step of (d') is repeatedly performed two or more times to form a plurality of additional first cell layers on the first cell layer.

4. The method of claim 1, wherein the steps of: (e) and (d) are sequentially performed, and further comprising a step of (e') selectively removing an additional second substrate by making the exposed second cell layer and an additional second cell layer provided on the additional second substrate come in contact with each other, and then making the additional second substrate come in contact with a solution containing the enzyme between the steps of: (e) and (d).

5. The method of claim 4, wherein the step of (e') is repeatedly performed two or more times to form a plurality of additional second cell layers on the second cell layer.

6. The method of claim 1, wherein the step of (d) makes the first substrate come in contact with a solution having a temperature lower than or equal to the melting point of the first substrate or a temperature at which the first substrate is changed to being hydrophilic, or allows the ambient temperature to be decreased to a temperature lower than or equal to the melting point of the first substrate or a temperature at which the first substrate is changed to being hydrophilic.

7. The method of claim 1, wherein the first cell layer and the second cell layer are formed by being cultured on the first substrate and the second substrate, respectively.

8. The method of claim 1, wherein the steps of: (a) and (c) are performed in an atmosphere of 35°C to 40°C.

9. The method of claim 1, wherein the first substrate comprises at least one of a polyphoshazene-based hydrogel, a Pluronic-based hydrogel, and poly(N-isopropylacrylamide).

10. The method of claim 1, wherein the first substrate is surface-treated with poly(N-isopropylacrylamide).

11. The method of claim 1, wherein the second substrate is a hydrogel substrate comprising carboxymethyl cellulose or alginate.

12. The method of claim 11, wherein the carboxymethyl cellulose or alginate is conjugated with tyramine.

13. The method of claim 1, wherein the enzyme is a carboxymethyl cellulose-degrading enzyme or an alginate-degrading enzyme.

14. The method of claim 1, wherein the first substrate and the second substrate have a predetermined pattern suitable for implementing the characteristics of the first cell layer and the second cell layer, respectively.

15. The method of claim 1, wherein the first substrate and the second substrate have a predetermined strength suitable for implementing the characteristics of the first cell layer and the second cell layer, respectively.

16. The method of claim 1, wherein the first cell layer and the second cell layer each comprise cells selected from the group consisting of mesenchymal stem cells (MSCs), myocyte precursor cells, myocytes, fibroblasts, chondrocytes, endothelial cells, epithelial cells, embryonic stem cells (ESCs), hematopoietic stem cells, anchorage-dependent cell precursors, induced pluripotent stem cells (iPSCs), and cardiomyocytes.

17. A multilayered cell sheet manufactured using the method of claim 1.
